(19)

**Europäisches Patentamt**
**European Patent Office**
**Office européen des brevets**

(11)  **EP 2 388 583 B1**

(12)  **EUROPÄISCHE PATENTSCHRIFT**

(45) Veröffentlichungstag und Bekanntmachung des Hinweises auf die Patenterteilung:
**08.06.2016  Patentblatt 2016/23**

(51) Int Cl.:
*G01N 33/28* (2006.01)      *G01N 11/10* (2006.01)
*F02D 41/14* (2006.01)      *F01L 9/02* (2006.01)

(21) Anmeldenummer: **11163106.5**

(22) Anmeldetag: **20.04.2011**

(54) **Verfahren sowie Steuereinrichtung zur Ermittlung einer Viskositäts-Kenngröße eines Öls**

Method and control device for determining a viscosity parameter of an oil

Procédé et dispositif de commande pour l'établissement d'une grandeur caractéristique de viscosité d'une huile

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.05.2010  DE 102010020754**

(43) Veröffentlichungstag der Anmeldung:
**23.11.2011  Patentblatt 2011/47**

(73) Patentinhaber: **Schaeffler Technologies AG & Co. KG**
**91074 Herzogenaurach (DE)**

(72) Erfinder:
- **Petz, Andreas**
  **85045 Ingolstadt (DE)**
- **Traversa, Piergiacomo**
  **90451 Nürnberg (DE)**
- **Di Pace, Marco**
  **91074 Herzogenaurach (DE)**
- **Tarassov, Ilia**
  **90491 Nürnberg (DE)**

(56) Entgegenhaltungen:
WO-A1-03/103837      DE-A1- 19 741 164
JP-A- 2004 092 593   US-A1- 2004 000 288
US-A1- 2004 244 742

**Beschreibung**

**Gebiet der Erfindung**

Die Erfindung betrifft ein Verfahren sowie eine Steuereinrichtung zur Ermittlung einer Viskositätskenngröße eines Öls in einer Maschine mit einer hydraulischen Steuerung, insbesondere eines Motoröls in einem Kraftfahrzeug vorzugsweise mit einer hydraulischen Steuerung der Gaswechselventile, wobei mit Hilfe eines Steuersignals eine hydraulische Komponente von einer ersten Position in eine zweite Position verstellt wird.

**Hintergrund der Erfindung**

Bei modernen Kraftfahrzeugen werden heutzutage aus Gründen der Schadstoffminimierung sowie der Verbrauchsreduzierung die sogenannten Gaswechselventile, also die Einlass- und/ oder Auslassventile für den Kraftfahrzeugmotor, lastabhängig gesteuert. Hierbei finden unterschiedliche Systeme Einsatz. Allen Systemen ist gemeinsam, dass die Schließ- und/ oder Öffnungszeiten der Gaswechselventile in Relation zur Kurbelwellenposition (Drehwinkel) lastabhängig verändert werden. Hier werden auch hydraulische Steuerungssysteme eingesetzt, bei denen mit Hilfe eines Hydraulikfluids, nämlich das Motoröl, eine Änderung der Schließ- und/ oder Öffnungszeiten des Gaswechselventils herbeigeführt wird.

So ist beispielsweise aus der EP 1 544 419 A1 ein erstes hydraulisches Steuerungssystem zu entnehmen, nämlich ein hydraulischer Nockenwellenversteller. Bei einem derartigen System wird mit Hilfe des Hydraulikfluids der Phasenwinkel zwischen der Kurbelwelle und der Nockenwelle eines Kraftfahrzeug-Motors verändert. Dies wird durch die variable Befüllung von Druckkammern einer Verstellvorrichtung erzielt. Zur Ansteuerung und Befüllung bzw. Entleerung der Druckkammern sind üblicherweise Steuerventile vorgesehen, die insbesondere als Magnetventile ausgebildet sind.

Aus dem Artikel "Elektrohydraulische Ventilsteuerung mit dem "MultiAir"-Verfahren" aus der motortechnischen Zeitschrift MTZ 12/2009 ist ein alternatives hydraulisches Steuersystem für die Ansteuerung der Gaswechselventile zu entnehmen. Bei dieser elektrohydraulischen Ventilsteuerung ist vorgesehen, dass die Bewegung der Nockenwelle über die Hydraulikflüssigkeit auf ein jeweiliges Gaswechselventil übertragen wird. Zur Steuerung ist ein insbesondere als Magnetventil ausgebildetes Steuer- oder Schaltventil vorgesehen. Im geschlossenen Zustand ist die Nockenwelle mit dem jeweiligen Gaswechselventil über ein sogenanntes hydraulisches Gestänge verbunden, so dass das Gaswechselventil einem Nocken der Nockenwelle zwangsweise folgt. Durch auch teilweises Öffnen des Schaltventils kann das Hydraulikfluid in einen Ausgleichs- oder Druckraum entweichen, so dass das Gaswechselventil von der Nockenbewegung entkoppelt ist. Hierdurch besteht die Möglichkeit, den Öffnungszeitpunkt, den Schließzeitpunkt sowie den Hub des Gaswechselventils innerhalb einer durch die Bewegung des Nocken vorgegebenen Hüllkurve zu variieren. Diese Variation kann zylinderselektiv erfolgen.

Im Hinblick auf die bei modernen Verbrennungsmotoren geforderte hohe Effizienz bei gleichzeitiger geringer Schadstoffemission ist eine exakte Ansteuerung der Gaswechselventile von entscheidender Bedeutung. Bei hydraulischen Systemen wirkt sich die Art der verwendeten Hydraulikflüssigkeit, also insbesondere die Qualität des eingesetzten Motoröls, auf den Betrieb wesentlich aus. Insbesondere ist die hydraulische Steuerung in ihrer Wirkungsweise empfindlich gegenüber Schwankungen bei der Viskosität des eingesetzten Öls.

Betriebsbedingt treten solche Schwankungen aufgrund der auftretenden unterschiedlichen Öltemperaturen auf. Wie aus dem Artikel "Elektrohydraulische Ventilsteuerung mit dem "MultiAir"-Verfahren" hervorgeht, wurden die temperaturabhängige Ölviskositätsschwankungen bisher in einem modellbasierten Steueralgorithmus berücksichtigt, der die Messwerte eines Öltemperatursensors berücksichtigt.

Diese temperaturabhängige und damit indirekte Bestimmung weist Nachteile auf und berücksichtigt beispielsweise keine Alterung des Motoröls oder einen Verschleiß der hydraulischen Komponenten.

Aus der JP 2004092593 ist ein Verfahren zu entnehmen, bei dem aus einer Antwortgeschwindigkeit der Gaswechselventile auf die Viskosität des Öls geschlossen wird.

Aus der US 2004/0000288 A1 ist ein System zu entnehmen, bei dem bei einem Einspritzventil ein Viskositätsparameter abgeleitet wird aus der Zeitdauer eines Steuersignals einer geschätzten Einspritzmenge eines Brennstoffs.

Auch aus der US 2004/0244742 A1 ist ein System zu entnehmen, bei dem eine Antwortzeit eines Ventils bestimmt ist auf der Basis der Viskosität eines Fluids. Aus der DE 197 41 164 A1 ist eine elektronisch gesteuerte und hydraulisch betätigte Treibstoffeinspritzvorrichtung zu entnehmen, bei der eine Ölviskositäts-Erfassungsvorrichtung vorgesehen ist.

Aus der WO 03/103837 A1 ist ein Verfahren und eine Vorrichtung zur Dosierung von Fluiden mit Hilfe eines Ventils zu entnehmen, wobei durch Auswertung der Schaltzeiten des Ventils die aktuelle Viskosität des Fluids ermittelt wird.

**Aufgabe der Erfindung**

Ausgehend hiervon liegt der Erfindung die Aufgabe zugrunde, ein verbessertes Verfahren sowie eine verbesserte Steuereinrichtung zur direkten Ermittlung einer Viskositätskenngröße für ein Öl anzugeben, wobei insbesondere auch Alterungs- und Verschleißeffekte berücksichtigt werden.

## Lösung der Aufgabe

Die Aufgabe wird gemäß der Erfindung gelöst durch ein Verfahren zur Ermittlung einer Viskositätskenngröße einer Hydraulikflüssigkeit in einer Maschine mit einer hydraulischen Steuerung gemäß Anspruch 1 sowie eine Steuereinrichtung gemäß Anspruch 12 wobei mit Hilfe eines üblicherweise elektrischen Steuersignals eine hydraulische Komponente von einer ersten Position in eine zweite Position verstellt wird. Die Zeitdauer, die die Komponente für den Wechsel von der ersten in die zweite Position benötigt, wird als Maß für die Viskositäts-Kenngröße herangezogen.

Diese Ausgestaltung beruht auf der grundsätzlichen Überlegung, dass der Bewegungsablauf einer hydraulischen Komponente des hydraulischen Systems bei einer Verstellbewegung der hydraulischen Komponente von einer ersten in eine zweite Position maßgeblich von der Qualität des verwendeten Öls und insbesondere von der Viskosität abhängt. Über die Viskosität wird quasi eine der Bewegung der hydraulischen Komponente entgegenwirkende Reibungskraft ausgeübt, so dass die Zeitdauer für die Verstellbewegung der hydraulischen Komponente einen Rückschluss auf die Viskosität zulässt.

Bei der hydraulischen Komponente handelt es sich hierbei insbesondere um eine Komponente, die kraftgesteuert, jedoch nicht mechanisch zwangsgesteuert von der ersten in die zweite Position wechselt, derart, dass unterschiedliche Reibungswiderstände zu unterschiedlichen Zeitdauern für die Verstellbewegung führen. Diese freie, nicht zwangsgeführte Bewegung wird auch als ballistische Bewegung bezeichnet. Die Kraft wird hierbei beispielsweise von einer Feder aufgebracht. Durch die entgegenwirkende viskositätsbedingte Reibungskraft des Öls (eventuell zusätzlich gegen die öldruckbedingte Kraft), variiert die Zeitdauer der Stellbewegungen in Abhängigkeit der Viskositäten.

Das Öl ist vorzugsweise ein Motoröl in einem Kraftfahrzeug, welches insbesondere mit einer hydraulischen Steuerung der Gaswechselventile, beispielsweise mit einem hydraulischen Nockenwellenversteller und insbesondere mit einer elektrohydraulischen Ventilsteuerung ausgestattet ist, wie sie beispielsweise aus der EP 1 544 419 A1 oder dem Artikel "Elektrohydraulische Ventilsteuerung mit dem "MultiAir"-Verfahren" zu entnehmen sind.

Unter Viskositäts-Kenngröße wird eine Kenngröße verstanden, die direkt mit der Viskosität korreliert ist. Die Viskositäts-Kenngröße ist hierbei insbesondere die Viskosität selbst, insbesondere die sogenannte kinematische Viskosität, oder eine aus der Viskosität abgeleitete Viskositätskennziffer wie beispielsweise die Veränderung der Viskosität, eine relative Viskosität etc.

Der besondere Vorteil bei der Auswertung der Zeitdauer der Verstellbewegung der hydraulischen Komponente während der ballistischen Bewegung ist darin zu sehen, dass - anders als bei einer rechnerischen Bestimmung aus der Öltemperatur - das tatsächliche durch die Viskosität bedingte Systemverhalten ausgewertet wird und somit eine direkte Kenngröße für die aktuelle Viskosität erhalten wird. Allein aus der Erfassung der Zeitdauer lässt sich ein sicherer und zuverlässiger Rückschluss auf den tatsächlichen Wert der Viskosität schließen. Im Vergleich zu der lediglich indirekten Bestimmung über die Temperatur des Motoröls werden bei diesem Verfahren direkt die Eigenschaften des Öls über den Bewegungsablauf der Komponente erfasst und somit können auch Alterungseffekte des Motoröls erfasst werden.

Weiterhin ist vorgesehen, dass eine Alterung der hydraulischen Komponente bei der Ermittlung der Viskositäts-Kenngröße ermittelt wird. Neben der Alterung des Öls nimmt auch eine Alterung des Schaltventils Einfluss auf die Zeitdauern. Die Alterung der hydraulischen Komponente wird aus der ermittelten Zeitdauer bei einem definierten Arbeitspunkt abgeleitet wird. Der definierte Arbeitspunkt ist insbesondere eine hohe Motoröltemperatur, vorzugsweise eine Temperatur > 80°C und insbesondere > 100°C. Durch die Ableitung eines Maßes für die Alterung aus der Zeitdauer wird - wie bereits bei der Bestimmung der Viskositäts-Kenngröße für das Öl - das tatsächliche Verhalten des Systems und nicht etwa nur ein postuliertes Verhalten des Systems ausgewertet. Damit ist die in diesem Sinne verstandene direkte Auswertung genauer und zuverlässiger. Die bevorzugte Auswertung bei einer hohen Motoröltemperatur gründet auf der Überlegung, dass bei diesen hohen Temperaturen der Einfluss der Viskosität des Öls auf die Zeitdauer zumindest vernachlässigbar ist aufgrund der bei hohen Temperaturen sehr geringen Viskosität, die gegen Null geht. Bei diesen hohen Temperaturen wird daher die Zeitdauer alleine durch die Ausgestaltung des Schaltventils bestimmt. Die Bestimmung der Alterung der Komponente aus der Zeitdauer kann auch ohne die Bestimmung der Viskositäts-Kenngröße durchgeführt werden und bildet einen eigenen, unabhängigen erfinderischen Aspekt.

Anhand der ermittelten Zeitdauer lässt sich daher ein Maß für den Alterungszustand ableiten. Dies ist wiederum sowohl absolut möglich, wenn in entsprechenden Zuordnungstabellen eine Zuordnung zwischen der jeweiligen Zeitdauer und dem Alterungszustand des Schaltventils hinterlegt ist. Alternativ kann auch ein relativer Alterungszustand durch Vergleich mit während des Betriebs zuvor erfassten Zeitdauern ermittelt werden.

Vorzugsweise wird als Maß für die Alterung einer Verschiebung einer Regressionsgeraden herangezogen, die sich aufgrund der linearen Regression für die Zuordnung zwischen der Zeitdauer zu der Viskosität ergibt, insbesondere gemäß folgender Formel:

$$\eta = m \cdot (t + t_{alt}) + b,$$

wobei $t_{alt}$ die durch die Alterung bedingte zusätzliche Zeitspanne ist.

Gemäß einer zweckdienlichen Ausgestaltung ist die hydraulische Komponente ein elektrisch steuerbares Steuer- oder Schaltventil, insbesondere ein Magnetventil. Derartige Ventile werden zum Schalten und Steuern des Öls beispielsweise für die Steuerung der Gaswechselventile eingesetzt. Das Ventil selbst bewegt sich insbesondere bei der ballistischen Bewegung zwischen einer Schließstellung und einer geöffneten Stellung, welche damit die ersten und zweiten Positionen des Schaltventils bilden. Da ein Verschlusselement des Schaltventils, wie beispielsweise ein Ventilteller, innerhalb des Strömungswegs des Öls geführt ist, wird die Stellbewegung des Verschlusselements durch die Eigenschaften des Öls beeinflusst. Bei einem Magnetventil erfolgt üblicherweise die Bewegung in die eine Stellung, vorzugsweise in die Schließstellung magnetkraftbetätigt, und nach Abschalten der magnetischen Kraft fährt das Ventil in die zweite Stellung, insbesondere in die Offenstellung federkraftbetätigt zurück.

Gemäß einer bevorzugten Weiterbildung wird die Zeit- oder Verstelldauer aus einem Steuersignal für das Schaltventil, insbesondere aus dessen Erregerstrom ermittelt. Es sind daher gemäß dieser bevorzugten Ausgestaltung keine zusätzlichen Messeinrichtungen wie Positionssensoren erforderlich. Die Ermittlung der Zeitdauer und damit die der Viskosität erfolgt daher alleine auswertetechnisch (softwaretechnisch) ohne dass zusätzliche Hardware-Komponenten erforderlich sind. Dadurch sind die Kosten für ein derartiges System gering gehalten.

Vorzugsweise wird zur Ermittlung der Zeitdauer eine Ausschaltzeit des Ventils herangezogen. Unter Ausschaltzeit wird hierbei insbesondere die Zeit verstanden, die zwischen einem Ausschaltsignal, insbesondere das Abschalten des Erregerstroms des Magnetventils und dem Erreichen einer insbesondere federkraftbetätigten Endstellung (Schließstellung) des Ventils.

Diese Auswertung beruht auf der Überlegung, dass nach dem Abschalten des Erregerstroms das Verschlusselement von der ersten in die zweite Position verfährt und hierdurch insbesondere durch die dabei auftretenden Beschleunigungen ein induktives Antwortsignal nach sich zieht, welches erfassbar ist. Da dieses Antwortsignal mit der Bewegung korreliert ist, lässt sich durch eine Auswertung dieses Antwortsignals (Stromsignal) eine Endstellung definieren und in reprodurzierbarer Weise ermitteln. Prinzipiell kann an Stelle des Erregerstroms auch ein Spannungssignal ausgewertet werden. Die Bestimmung und Auswertung der Ausschaltzeit des Schaltventils erfolgt beispielsweise nach der Methode, wie sie in der EP 1 533 506 A2 beschrieben ist.

Vorzugsweise wird anhand der ermittelten Zeitdauer unmittelbar auf die Viskosität des Öls aufgrund einer bekannten Zuordnung zurückgeschlossen. Hierzu ist vorzugsweise in einer Auswerteeinheit eine Zuordnung zwischen den Werten für die Zeitdauer und den Werten für die Viskositäts-Kenngröße, insbesondere für die absoluten Viskositäten hinterlegt. Der aktuelle Wert der Viskosität wird daher insbesondere durch einen einfachen Vergleich ermittelt. Die Zuordnung kann hierbei beispielsweise im Rahmen einer Tabelle, einer mathematischen Funktion oder einem Kennfeld erfolgen. Hierbei sind vorzugsweise für unterschiedliche Ölarten unterschiedliche Zuordnungen hinterlegt.

In zweckdienlicher Ausgestaltung wird diese Zuordnung in Testläufen im Neuzustand des Systems in Abhängigkeit verschiedenster Betriebsparameter bzw. Arbeitspunkte ermittelt und die so gewonnenen Ergebnisse in der Auswerteeinheit hinterlegt. Hierbei können umfangreiche Kennlinienfelder vorgesehen sein, die beispielsweise die Einwirkung von unterschiedlichen Ölarten, den Einfluss der Temperatur, etc. auf die jeweilige Viskosität abbilden.

Vorzugsweise erfolgt die Zuordnung zwischen der Zeitdauer und der Viskosität mittels einer linearen Regression. Untersuchungen haben gezeigt, dass zwischen der Zeitdauer und dem absoluten Wert der Viskosität ein einfacher linearer Zusammenhang besteht gemäß folgender Gleichung:

$$\eta = m \cdot t + b \, ,$$

wobei

$\eta$ die Viskosität des Öls beispielsweise in [$m^2$ / $s$], $t$ die ermittelte Zeitdauer (Ausschaltzeit) und $m$, $b$ das System, insbesondere das Magnetventil charakterisierende Konstanten sind.

In bevorzugter Weiterbildung ist vorgesehen, dass im Laufe des Betriebs die Zeitdauer wiederholt ermittelt wird und auf eine Änderung überprüft wird. Da die Zeitdauer stark von den Betriebsbedingungen, insbesondere Temperatur des Öls abhängt, werden hierzu nur die Zeitdauern eines bestimmten Arbeitspunktens miteinander verglichen, insbesondere die Zeitdauern bei einer gleichen Öltemperatur. Durch diesen Vergleich wird daher eine Veränderung der Viskositäts-Kenngröße ermittelt. Unter "im Laufe des Betriebs" ist hier der normale bestimmungsgemäße Betrieb der Maschine zu verstehen. Beim Kraftfahrzeug ist dies der normale Fahrbetrieb über Tage, Wochen, Monate und Jahre. Es wird daher vorzugsweise eine dauernde Erfassung der Zeitdauer über die gesamte Lebensdauer durchgeführt. Die Erfassung der Zeitdauer erfolgt hierbei vorzugsweise in wiederkehrenden Abständen, ausgelöst beispielsweise durch bestimmte Ereignisse, wie beispielsweise starten des Motors. Daneben kann jedoch auch eine Auswertung in kürzeren regelmäßigen Zeitabständen, beispielsweise in Abhängigkeit der Motorumdrehungen erfolgen.

Anhand dieser Änderung wird in zweckdienlicher Weiterbildung auf eine Alterung des Motoröls und/oder der hydraulischen Komponente, also des Schaltventils zurückgeschlossen. In einer alternativen Ausgestaltung wird alleine anhand der ermittelten Zeitdauer auch auf die Alterung zurückgeschlossen. So führt beispielsweise eine Alterung des Motoröls in der Regel zu einer Erhöhung der Viskosität und damit zu einer Erhöhung der Zeitdauer bei gleicher Temperatur. Es lassen sich daher prinzipiell für unterschiedliche Alterungszustände unterschiedliche Kurven hinterlegen, so dass allein aufgrund der Messung der Zeitdauer (bei definierter Temperatur) ein Rückschluss auf den Zustand des Motoröls im Hinblick auf eine Erhöhung der Viskosität möglich ist.

Die ermittelte Viskositäts-Kenngröße, insbesondere auch die ermittelte Änderung der Kenngröße, vorzugsweise die absolute Viskosität, wird in zweckdienlicher Weise wahlweise oder in Kombination für unterschiedliche abgeleitete Maßnahmen herangezogen. Gemäß einer bevorzugten ersten Alternative wird hieraus die eingesetzte Ölqualität bestimmt und abgeleitet. Durch Messungen beispielsweise bei unterschiedlichen Arbeitspunkten (Temperaturen) lässt sich das Öl insgesamt sehr gut charakterisieren. Insbesondere besteht die Möglichkeit, aufgrund der gemessenen Zeitdauern insbesondere in Kombination mit einer Hinterlegung einer Vielzahl von Kennlinienfeldern für unterschiedliche Ölarten und Ölqualitäten, eine Zuordnung zu einer jeweiligen Ölqualität vorzunehmen. Ergänzend ist vorzugweise die Erkennung von unzulässigen Ölqualitäten vorgesehen, und bei Bedarf die Ausgabe eines Warnsignals, beispielsweise eine Aufforderung zu einem Motorölwechsel.

Weiterhin ist vorzugsweise die Bestimmung des Ölwechselintervalls bzw. des nächsten Zeitpunkts für einen fälligen Ölwechsel vorgesehen. Aufgrund der Erfassung des tatsächlichen Zustands des Öls wird daher zuverlässig und sicher frühzeitig erkannt, ob das Öl noch den Anforderungen genügt, so dass zuverlässig ein Zeitpunkt für den nächsten Ölwechsel bestimmt werden kann. Durch die Erkennung der Motorölqualität ist generell daher auch das Ölwechselintervall bestimmbar.

Weiterhin wird vorzugsweise eine Diagnose der hydraulischen Komponente, also insbesondere des Magnetventils vorgenommen, und beispielsweise im Hinblick auf einen notwendigen Austausch überprüft, auf den im Rahmen der Auswertung und Diagnose auch vorzugsweise hingewiesen wird.

Weiterhin wird vorzugsweise eine Diagnose der hydraulischen Komponente, also insbesondere des Magnetventils vorgenommen, und beispielsweise im Hinblick auf einen notwendigen Austausch überprüft, auf den im Rahmen der Auswertung und Diagnose auch vorzugsweise hingewiesen wird.

Schließlich wird in besonders zweckdienlicher Ausgestaltung anhand der ermittelten Größen Einfluss auf die Ventilsteuerung genommen. Zum einen werden beispielsweise bei einer erfassten Alterung des Motoröls, d.h. bei einer erfassten Erhöhung der Viskosität, die Zeitpunkte für die Ansteuerung des Schaltventils variiert und den neuen Gegebenheiten angepasst, um eine möglichst exakte Gasventilsteuerung entsprechend den jeweiligen Anforderungen aufrecht zu erhalten. Darüber hinaus wird in bevorzugter Alternative die gesamte hydraulische Steuerung auf Grundlage der ermittelten Kenngröße für die Viskosität vorgenommen, und nicht mehr wie bisher üblich anhand der aktuellen Öltemperatur. Die gesamte Steuerung berücksichtigt daher den tatsächlichen Zustand des Öls.

Gemäß einer bevorzugten Ausgestaltung ist darüber hinaus vorgesehen, dass ein Warnsignal abgegeben wird, wenn die ermittelte Ölviskosität einen Störungsfreien Betrieb des Motors nicht zulässt. Weiterhin ist vorgesehen, dass sogar ein Starten des Motors unterbunden wird, um beispielsweise aufgrund einer zu hohen Viskosität und einer damit einhergehenden mangelnden Schmierung des Motors einen Motorschaden zu verhindern. Dies ist beispielsweise im Winter von Vorteil, wenn aufgrund der Außentemperaturen die anfängliche Viskosität beim Kaltstart zu hoch ist. Aufgrund des in einem vorhergehenden Betriebszyklus ermittelten Ölzustands lässt sich daher eine minimale Außentemperatur bestimmen, die nicht unterschritten werden darf.

**Kurze Beschreibung der Zeichnung**

Ein Ausführungsbeispiel der Erfindung wird im Folgenden anhand der Zeichnung näher erläutert. Es zeigen:

Fig. 1     in einer schematischen und stark vereinfachten ausschnittsweisen Darstellung die prinzipielle Funktionsweise einer elektrohydraulischen Steuerung eines Gasventils in einem Kraftfahrzeugmotor,

Fig. 2     eine schematische, stark vereinfachte Darstellung des Verlaufs eines Steuer- oder Erregerstroms für ein als Magnetventil ausgebildetes Schaltventil,

Fig. 3     ein Diagramm, bei dem die kinematische Viskosität eines Öls gegenüber der Ausschaltzeit eines Magnetventils für ein System im Neuzustand (neues Öl und neues Schaltventil) aufgetragen ist,

Fig. 4     ein zu Fig. 3 vergleichbares Diagramm, wobei eine zusätzliche Regressionsgerade für ein System mit einem gealterten Schaltventil eingetragen ist,

Fig. 5     ein schematisches stark vereinfachtes Diagramm, bei dem die Zeitdauer bzw. Viskosität gegenüber der Öltemperatur für zwei verschiedene Ölarten bzw. Ölqualitäten aufgetragen ist, sowie

Fig. 6     ein schematisches Diagramm, bei dem die Zeitdauer bzw. Viskosität gegenüber der Öltemperatur angetragen ist für ein Öl im Neuzustand und im gealterten Zustand.

In den Figuren sind gleich wirkende Teile mit den gleichen Bezugszeichen versehen.

## Ausführliche Beschreibung der Zeichnung

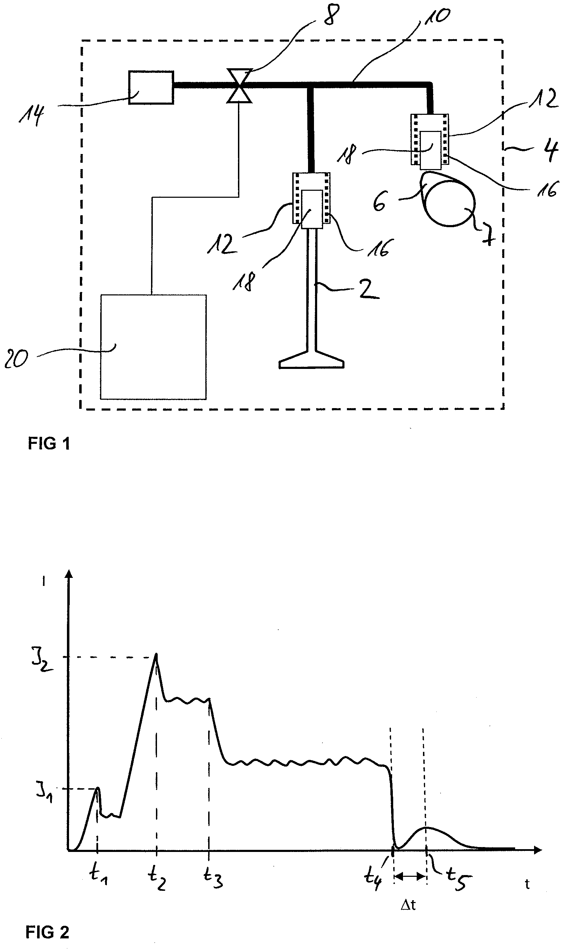

Bei einem elektrohydraulischen Steuerungssystem zur hydraulischen Steuerung eines Gaswechselventils 2 in einem in Fig. 1 durch gestrichelte Umrandung angedeuteten Kraftfahrzeug 4 umfasst ein hydraulisches System, welches eine Bewegung eines Nocken 6 einer Nockenwelle 7 auf ein jeweiliges Gaswechselventil 2 mittels einer Hydraulikflüssigkeit, nämlich dem Motoröl überträgt. Die elektrohydraulische Ventilsteuerung ist an sich bekannt. Wesentliches Merkmal ist ein insbesondere als Magnetventil 8 ausgebildetes Schaltventil, welches in eine Hydraulikleitung 10 geschaltet ist. Die Hydraulikleitung 10 ist über einen Hydraulikzylinder 12 einerseits mit dem Nocken 6 und andererseits mit dem Gaswechselventil 2 verbunden. Über das Magnetventil 8 lässt sich die Hydraulikleitung 10 zu einem Ausgleichs- oder Druckraum 14 absperren. Innerhalb der Hydraulikzylinder 12 sind Kolben 18 beispielsweise gegen die Kraft einer Feder 16 beweglich gelagert. Bei einer Rotation des Nockens 6 folgt der Kolben 18 des zugeordneten Hydraulikzylinders 12 der Nockenbewegung. Bei geschlossenem Magnetventil 8 wirkt das hydraulische System nach Art eines hydraulischen Gestänges, so dass der Kolben 18 in dem dem Gaswechselventil 2 zugeordneten Hydraulikzylinder 12 der Bewegung des Nockens 6 unmittelbar folgt.

Durch Öffnen des Magnetventils 8 kann das Öl in den Druckraum 14 ausweichen, so dass die Bewegung des Gaswechselventils 2 von der Bewegung des Nockens 6 entkoppelt ist.

Das Magnetventil 8 ist mit einer Steuer- oder Auswerteeinheit 20 verbunden. Die Steuereinheit 20 ist beispielsweise in der Motorsteuerung integriert. Über die Steuereinheit 20 wird das Magnetventil mit einem Steuersignal beaufschlagt. Im Ausführungsbeispiel ist dies ein Erregerstrom I für eine Magnetspule des Magnetventils 8.

Üblicherweise ist das Magnetventil 8 im nicht aktivierten Zustand offen, so dass die Hydraulikleitung 10 zum Druckraum 14 hin frei ist. Im aktivierten Zustand, wenn also das Magnetventil 8 mit einem ausreichenden Erregerstrom I beaufschlagt wird, befindet sich das Magnetventil 8 in seiner Schließstellung. Das Magnetventil 8 weist hierbei einen an sich bekannten und typischen Aufbau für ein Magnetventil auf. Ein Anker wird in Schließ- bzw. Öffnungsrichtung durch den durch eine Elektrospule gebildeten Magneten betätigt. An diesem Anker ist ein Verschlusselement zum Verschließen der Hydraulikleitung 10 angeordnet. Üblicherweise wirkt die Magnetkraft gegen eine Federkraft einer im Magnetventil 8 angebrachten Feder, die das Magnetventil 8 im nicht aktiven Zustand in seine Ausgangslage, insbesondere seine Offenstellung drückt.

Der Erregerstrom I zeigt üblicherweise einen typischen Verlauf wie er in Fig. 2 dargestellt ist. Üblicherweise wird die Spule zunächst mit einem Einschaltstrom $I_1$ zu einer Zeit $t_1$ beaufschlagt. Dieser Einschaltstrom $I_1$ führt lediglich zu einer Vormagnetisierung nicht jedoch zu einer Bewegung des Verschlusselements. Zum Aktivieren, also Schließen des Ventils 8 wird zum Zeitpunkt $t_2$ dieses mit einem Schließstrom $I_2$ beaufschlagt. Zu diesem Zeitpunkt bewegt sich das Verschlusselement in seine Schließstellung. Aufgrund einer induktiven Antwort fällt der Schließstrom etwas ab. Nach dem Schließen wird üblicherweise zu einem Zeitpunkt $t_3$ der Strom auf einen Haltestrom $I_3$ reduziert.

Zum Öffnen des Ventils wird zu einem Zeitpunkt $t_4$ die Stromzufuhr abgeschaltet. Aufgrund der Feder verschiebt sich das Verschlusselement in Richtung zur Offenstellung. Hierbei wird wiederum eine induktive Antwort erzeugt, die sich im Nachgang zum Zeitpunkt $t_4$ in einem Stromimpuls äußert, wie er aus Fig. 2 zu entnehmen ist. Der Verlauf dieses Stromimpulses korreliert zu der Bewegung des Verschlusselements des Magnetventils 8. Aus dem Verlauf des Stromimpulses ist eine definierte Position des Verschlusselements, insbesondere seine Offenstellung eindeutig ableitbar. Diese wird im Ausführungsbeispiel zum Zeitpunkt $t_5$ erreicht.

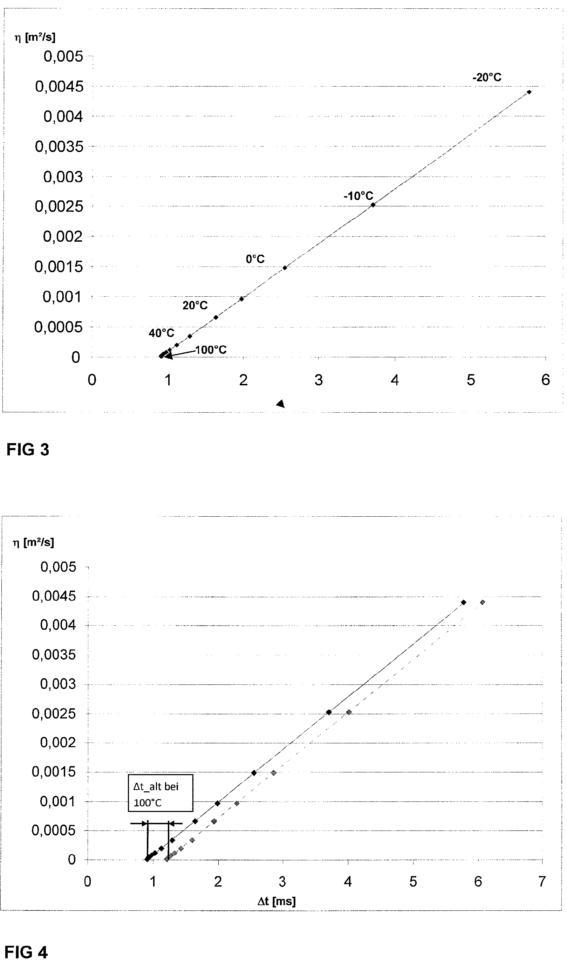

Die Zeitpunkte $t_4$ sowie $t_5$ entsprechen daher einer ersten und einer zweiten Position des Magnetventils 8. Die Zeitdauer $\Delta t$ zwischen $t_4$ und $t_5$ repräsentiert die Ausschaltzeit für den Schaltvorgang und damit den Verstellvorgang des Magnetventils. Die Zeitdauer $\Delta t$ ist unmittelbar mit der Viskosität $\eta$ des verwendeten Öls verknüpft. Untersuchungen haben gezeigt, dass hierbei ein linearer Zusammenhang zwischen der Zeitdauer $\Delta t$ und der kinematischen Viskosität $\eta$ besteht, wie er in Fig. 3 beispielhaft dargestellt ist. Jede Zeitdauer $\Delta t$ ist eindeutig ein spezieller Wert für die Viskosität $\eta$ zugewiesen. Die einzelnen durch Punkte hervorgehobenen Messpunkte lassen sich durch eine Regressionsgerade verbinden. Dies lässt sich allgemein nach der Gleichung bestimmen:

$$\eta = m\, \Delta t + b.$$

Hierbei sind:

$\eta$ die kinematische Viskosität,
$\Delta t$ die Zeitdauer bzw. Ausschaltzeit des Magnetventils 8,
m, b magnetventilspezifische Konstanten, die das Magnetventil charakterisieren.

Wie in dem Diagramm gemäß Fig. 3 dargestellt, entsprechen die einzelnen Messpunkte bestimmten Temperaturen des Öls bei Verwendung des identischen Öls. Die einzelnen Temperaturwerte definieren hierbei einen bestimmten Arbeitspunkt des Kraftfahrzeugmotors.

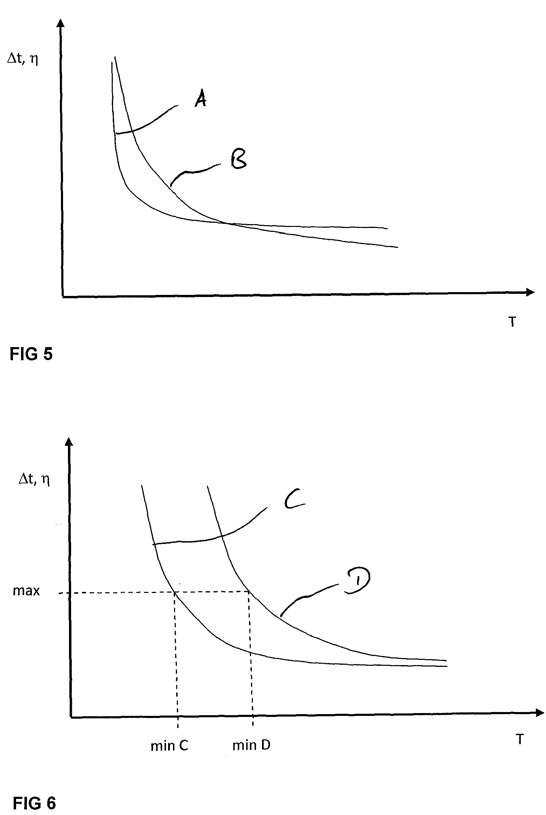

Der lineare Zusammenhang zwischen Viskosität η und Zeitdauer Δt ist unabhängig von der Wahl des Motoröls und auch dessen Alterungszustand. Unterschiedliche Motoröle bzw. unterschiedliche Alterungszustände eines Öls äußern sich darin, dass bei definiertem Arbeitspunkt, also bei definierter Temperatur, die Schaltzeiten Δt bzw. damit einhergehend die Viskositäten Δ unterschiedlich sind. Dies wird nachfolgend noch im Zusammenhang mit den Fig. 5 und 6 erläutert.

Beim Diagramm gemäß Fig. 4 ist eine Alterung des Magnetventils 8 berücksichtig. Untersuchungen haben gezeigt, dass sich dies in einer Verschiebung der Regressionsgeraden äußert. Die bei einem gealterten Magnetventil 8 relevante Regressionsgerade lässt sich durch folgende Gleichung ausdrücken:

$$\eta = m \, (\Delta t + \Delta t_{alt}) + b,$$

wobei

$\Delta t_{alt}$ eine Veränderung der Zeitdauer Δt aufgrund der Alterung des Magnetventils 8 ist.

Der Wert von $\Delta t_{alt}$ wird hierbei bei hohen Öltemperaturen > 80°C, beispielsweise ≥ 100°C erfasst. Bei diesen Temperaturen wird die Zeitdauer Δt aufgrund der niedrigen Viskosität des Öls, die hier nahezu Null erreicht, allein durch die Eigenschaften des Magnetventils 8 bestimmt. Im Diagramm der Fig. 3 gibt daher die linke Gerade die Regressionsgerade bei einem neuen Magnetventil 8 und die nach rechts verschobene Regressionsgerade die Regressionsgerade mit gealtertem Magnetventil 8 an.

Sobald eine derartige Verschiebung aufgrund einer Alterung des Magnetventils 8 festgestellt wird, wird die verschobene Regressionsgerade für die Zuordnung zwischen der Zeitdauer Δt und der Viskosität η herangezogen.

In Fig. 5 ist stark vereinfacht ein Diagramm dargestellt, bei dem die Temperatur gegenüber der Zeitdauer Δt und korrespondierend hierzu von η aufgetragen ist. Identisches gilt für das Diagramm gemäß Fig. 6.

Fig. 5 zeigt zwei Kurven A, B für zwei unterschiedliche Motorölarten bzw. Qualitäten.

Zur Ermittlung der eingesetzten Motorölqualität bzw. zur Ermittlung der Art des verwendeten Öls wird insbesondere folgendermaßen vorgegangen:

Für unterschiedliche Öltemperaturen T werden die Zeitdauern Δt erfasst. Durch die so ermittelten Messpunkte wird beispielsweise eine gefittete Kurve gelegt. Die gemessene Zuordnung zwischen Temperatur und Zeitdauer Δt wird mit bekannten, in der Steuereinheit 20 hinterlegten Zuordnungen, beispielsweise Kennlinienfeldern verglichen. Anhand einer Übereinstimmung bei dem Vergleich mit einem bekannten Kennlinienfeld wird dann das aktuell verwendete Öl einem bekannten Öl bestimmter Qualität zugeordnet. Prinzipiell ist die Bestimmung der Qualität jedoch allein auch aufgrund der Messwerte ohne Vergleich mit den hinterlegten Kennlinienfeldern möglich.

Das Diagramm gemäß Fig. 6 zeigt schematisch und stark vereinfacht den Effekt der Alterung des Motoröls. Die beiden Kurven C, D geben einerseits die Temperaturabhängigkeit für ein neues Öl (Kurve C) und andererseits des gleichen, gealterten Öls (Kurve D) wieder. Die Alterung des Öls schlägt sich in einer Zunahme der Viskosität η und damit der Zeitdauer Δt bei gleicher Temperatur T nieder.

In der Fig. 6 ist beispielhaft ein maximal zulässiger Wert max für die Zeitdauer Δt bzw. für die Viskosität η eingezeichnet. Diesem Maximalwert max sind für die beiden Kurven unterschiedliche minimale Temperaturen T minC, minD zugeordnet. Wie aus dem Diagramm abzulesen ist, ist mit dem neuen Öl eine geringere Minimaltemperatur minC als mit dem gealterten Öl (minD) zulässig.

Dies wird beispielsweise herangezogen, um bei Unterschreiten der minimal zulässigen Temperatur minC, minD ein Warnsignal abzugeben oder im Extremfall ein Starten des Motors zu unterbinden.

Die Ermittlung der gealterten Kurve D erfolgt auf ähnliche Weise wie zu Fig. 5 beschrieben, indem bei verschiedenen Temperaturen T die Zeitdauern Δt ermittelt werden. Durch laufende bzw. wiederkehrende Messungen während des Betriebs wird die zunehmende Alterung erfasst. Ein Motorölwechsel wird durch eine abrupte Änderung des Kurvenverlaufs erfasst.

Das hier beschriebene Verfahren zeichnet sich dadurch aus, dass die Zeitdauer Δt als Kenngröße für die aktuelle Viskosität η des Motoröls erfasst und ausgewertet wird. Es wird daher der tatsächliche Ist-Systemzustand des hydraulischen Systems ermittelt.

Von besonderer Bedeutung ist weiterhin, dass zur Ermittlung der Zeitdauer Δt die Zeitdauer für die Stellbewegung des Magnetventils ausgewertet wird, und dass dies insbesondere über eine Auswertung des Steuersignals I für das Magnetventil 8 erfolgt.

Ein weiterer wesentlicher Gesichtspunkt ist darin zu sehen, dass die Alterung des Schaltventils 8 berücksichtigt wird. Von besonderer Bedeutung hierbei ist wiederum, dass auch die Alterung des Schaltsignals durch eine Auswertung der Zeitdauer Δt erhalten wird.

Allgemein wird daher die Übertragung oder Relation zwischen der elektrischen Ansteuerung der hydraulischen Komponente (Magnetventil 8) und der hierdurch ausgelösten hydraulischen Aktion (Schließen/Öffnen) ausgewertet und die Viskosität η abgeleitet. Unabhängig von der elektrischen Ansteuerung des Magnetventils bestimmt diese sich in der Reaktionszeit und damit der Zeitdauer Δt niederschlagende Korrelation durch eine Änderung der Funktion des Magnetventils 8 (Alterung, mechanischer Verschleiß) und durch eine Änderung der Viskosität η der Hydraulikflüssigkeit. Die Änderung der Viskosität ist hierbei bedingt durch einen Verschleiß bzw. Alterung des Öls bzw. durch die aktuelle Temperatur der Hydraulikflüssigkeit.

Der besondere Vorteil der Ermittlung der tatsächlichen Viskosität η besteht insbesondere bei einem zeitkritischen und emissionsrelevanten System wie bei einer elektrohydraulischen Ventilsteuerung darin, dass ein derartiges hydraulisches Steuersystem für die Ansteuerung der Gaswechselventile unabhängig von der Alterung des Öls ohne Beeinträchtigung der Funktion und der Abgaswerte betrieben werden kann.

Zweckdienlicherweise wird hierzu aufgrund der gewonnenen Erkenntnisse die Steuerung der Gaswechselventile aktiv beeinflusst. Vorzugsweise wird die gesamte Steuerung der Gaswechselventile mit Hilfe des vorliegenden Verfahrens basierend auf der tatsächlich bestehenden Viskosität η (und nicht wie bisher mittelbar über die Temperatur) durchgeführt. Der Steuerparameter ist daher die über die Zeitdauer Δt ermittelte tatsächliche Viskosität.

**Liste der Bezugszahlen**

2 Gaswechselventil
4 Kraftfahrzeug
6 Nocken
7 Nockenwelle
8 Magnetventil
10 Hydraulikleitung
12 Hydraulikzylinder
14 Druckraum
16 Feder
18 Kolben
20 Steuereinheit

**Patentansprüche**

1. Verfahren zur Ermittlung einer Viskositäts-Kenngröße (η) einer Hydraulikflüssigkeit in einer Maschine mit einer hydraulischen Steuerung, insbesondere eines Motoröls in einem Kraftfahrzeug (4) mit einer hydraulischen Steuerung der Gaswechselventile (2), wobei mit Hilfe eines Steuersignals (3) eine hydraulische Komponente (8) von einer ersten Position in eine zweite Position verstellt wird, wobei eine Zeitdauer (Δt), die die Komponente (8) für den Wechsel von der ersten in die zweite Position benötigt, als Maß für die Viskositäts-Kenngröße (η) herangezogen wird, **dadurch gekennzeichnet, dass** ergänzend anhand der Zeitdauer (Δt) bei einem Arbeitspunkt auf eine Alterung der hydraulischen Komponente (8) geschlossen wird.

2. Verfahren nach Anspruch 1, **dadurch gekennzeichnet, dass** die Alterung der hydraulischen Komponente (8) aus der ermittelten Zeitdauer (Δt) bei einer bestimmten Temperatur (T) des Öls, vorzugsweise bei einer Temperatur größer 80°C abgeleitet wird.

3. Verfahren nach Anspruch 1 oder 2, **dadurch gekennzeichnet, dass** eine Zuordnung zwischen der Zeitdauer (Δt) und der Viskosität (η) der Hydraulikflüssigkeit durch eine mittels linearer Regression ermittelten Regressionsgeraden erfolgt und als Maß für die Alterung eine Verschiebung der Regressionsgeraden gegenüber einem nicht gealterten Zustand herangezogen wird.

4. Verfahren nach einem der vorhergehenden Ansprüche, **dadurch gekennzeichnet, dass** die hydraulische Komponente ein elektrisch steuerbares Schaltventil, insbesondere ein Magnetventil (8) ist.

5. Verfahren nach Anspruch 4, **dadurch gekennzeichnet, dass** zur Ermittlung der Zeitdauer (Δt) ein Steuersignal (I) für das Schaltventil (8), insbesondere dessen Erregerstrom, ausgewertet wird.

6. Verfahren nach Anspruch 4 oder 5, **dadurch gekennzeichnet, dass** eine Schaltzeit des Schaltventils (8) zwischen dem Steuersignal (I) und dem Erreichen einer Endstellung des Schaltventils (8) als Zeitdauer (Δt) herangezogen wird.

7. Verfahren nach einem der vorhergehenden Ansprüche, **dadurch gekennzeichnet, dass** anhand der Zeitdauer (Δt) unmittelbar auf die Viskosität (η) der Hydraulikflüssigkeit aufgrund einer bekannten Zuordnung zurückgeschlossen wird.

8. Verfahren nach Anspruch 7, **dadurch gekennzeichnet, dass** die Zuordnung zwischen der Zeitdauer (Δt) und der Viskosität (η) mittels linearer Regression erfolgt.

9. Verfahren nach einem der vorhergehenden Ansprüche, **dadurch gekennzeichnet, dass** die Zeitdauer (Δt) wiederholt bei einem bestimmten Arbeitspunkt, insbesondere bei einer bestimmten Temperatur (T) der Hydraulikflüssigkeit ermittelt wird und auf eine Änderung überprüft wird.

10. Verfahren nach einem der vorhergehenden Ansprüche, **dadurch gekennzeichnet, dass** anhand der Zeitdauer (Δt) bei einem bestimmten Arbeitspunkt auf eine Alterung der Hydraulikflüssigkeit geschlossen wird.

11. Verfahren nach einem der vorhergehenden Ansprüche, **dadurch gekennzeichnet, dass** die ermittelte Viskositäts-Kenngröße (η) ausgewertet und wahlweise oder in Kombination herangezogen wird für

- eine Ermittlung eines Maßes für eine Ölqualität
- eine Erkennung von unzulässigen Motorölen
- eine Bestimmung eines Ölwechselintervalls
- eine Diagnose der hydraulischen Komponente (8)
- die Ventilsteuerung.

12. Steuereinrichtung (20) zur Ermittlung einer Viskositäts-Kenngröße (η) einer Hydraulikflüssigkeit in einer Maschine mit einer hydraulischen Steuerung, insbesondere eines Motoröls in einem Kraftfahrzeug mit einer hydraulischen Steuerung der Gaswechselventile, wobei mit Hilfe eines Steuersignals (I) eine hydraulische Komponente (8) von einer ersten Position in eine zweite Position verstellt wird, wobei die Steuereinrichtung (20) derart ausgebildet ist, dass eine Zeitdauer (Δt), die die Komponente (8) für den Wechsel von der ersten in die zweite Position benötigt, als Maß für die Viskositäts-Kenngröße (η) herangezogen wird, **dadurch gekennzeichnet, dass** ergänzend anhand der Zeitdauer (Δt) bei einem Arbeitspunkt auf eine Alterung der hydraulischen Komponente geschlossen wird.

**Claims**

1. Method for determining a characteristic viscosity variable (η) of a hydraulic liquid in a machine with hydraulic control, in particular of an engine oil in a motor vehicle (4) with hydraulic control of the gas exchange valves (2), with a hydraulic component (8) being adjusted from a first position to a second position with the aid of a control signal (3), wherein a time period (Δt) which the component (8) requires for the changeover from the first position to the second position is used as a measure of the characteristic viscosity variable (η), **characterized in that**, in addition, a conclusion can be drawn about aging of the hydraulic component (8) at an operating point on the basis of the time period (Δt).

2. Method according to Claim 1, **characterized in that** aging of the hydraulic component (8) is derived from the determined time period (Δt) at a specific temperature (T) of the oil, preferably at a temperature of greater than 80°C.

3. Method according to Claim 1 or 2, **characterized in that** an association between the time period (Δt) and the viscosity (η) of the hydraulic liquid is made by a regression line which is determined by means of linear regression, and a shrift in the regression line in relation to an unaged state is used as a measure of aging.

4. Method according to one of the preceding claims, **characterized in that** the hydraulic component is an electrically controllable switching valve, in particular a solenoid valve (8).

5. Method according to Claim 4, **characterized in that** a control signal (I) for the switching valve (8), in particular the field current of said switching valve, is evaluated in order to determine the time period (Δt).

6. Method according to Claim 4 or 5, **characterized in that** a switching time of the switching valve (8) between the control signal (I) and an end position of the switching valve (8) being reached is used as the time period (Δt).

7. Method according to one of the preceding claims, **characterized in that** a direct conclusion is drawn about the viscosity (η) of the hydraulic liquid on the basis of a known association using the time period (Δt).

8. Method according to Claim 7, **characterized in that** the association between the time period (Δt) and the viscosity (η) is made by means of linear regression.

9. Method according to one of the preceding claims, **characterized in that** the time period (Δt) is repeatedly determined at a specific operating point, in particular at a specific temperature (T) of the hydraulic liquid, and a check for a change is made.

10. Method according to one of the preceding claims, f **characterized in that** a conclusion can be drawn about aging of the hydraulic liquid at a specific operating point on the basis of the time period (Δt).

11. Method according to one of the preceding claims, **characterized in that** the determined characteristic viscosity variable (η) is evaluated and used selectively or in combination for

- determining a measure of an oil quality
- identifying impermissible engine oils
- establishing an oil change interval
- diagnosing the hydraulic component (8)
- valve control.

12. Control device (20) for determining a characteristic viscosity variable (η) of a hydraulic liquid in a machine with hydraulic control, in particular of an engine oil in a motor vehicle with hydraulic control of the gas exchange valves, with a hydraulic component (8) being adjusted from a first position to a second position with the aid of a control signal (I), wherein the control device (20) is formed in such a way that a time period (Δt) which the component (8) requires for the changeover from the first position to the second position is used as a measure of the characteristic viscosity variable ($\eta$), **characterized in that**, in addition, a conclusion can be drawn about aging of the hydraulic component at an operating point on the basis of the time period ($\Delta t$).

## Revendications

1. Procédé de détermination d'une grandeur caractéristique de viscosité ($\Delta$) d'un liquide hydraulique dans une machine équipée d'une commande hydraulique, notamment d'une huile moteur dans un véhicule automobile (4) équipé d'une commande hydraulique des soupapes d'échange de gaz (2), un composant hydraulique (8) étant déplacé d'une première position dans une deuxième position à l'aide d'un signal de commande (3), une durée ($\Delta t$) nécessaire pour faire passer le composant (8) de la première dans la deuxième position étant déduite, cette durée servant de mesure pour la grandeur caractéristique de viscosité ($\eta$), **caractérisé en ce que** l'on déduit de façon complémentaire à l'aide de la durée ($\Delta t$) pour atteindre le point de travail un vieillissement du composant hydraulique (8).

2. Procédé selon la revendication 1, **caractérisé en ce que** le vieillissement du composant hydraulique (8) est déduit à partir de la durée ($\Delta t$) déterminée à une température (T) définie de l'huile, de préférence à une température supérieure à 80 °C.

3. Procédé selon la revendication 1 ou 2, **caractérisé en ce qu'**une association entre la durée ($\Delta t$) et la viscosity ($\eta$) du liquide hydraulique se produit par le biais d'une régression linéaire des droites de régression déterminées et qu'on en déduit, en l'utilisant comme mesure pour le vieillissement, un décalage des droites de régression par rapport à un état non vieilli.

4. Procédé selon l'une quelconque des revendications précédentes, **caractérisé en ce que** le composant hydraulique est une soupape de commutation à commande électrique, notamment une électrovanne (8).

5. Procédé selon la revendication 4, **caractérisé en ce qu'**on analyse un signal de commande (I) de la soupape de commutation (8), notamment son courant d'excitation, pour déterminer la durée ($\eta t$).

6. Procédé selon la revendication 4 ou 5, **caractérisé en ce qu'**on déduit un moment de commutation de la soupape de commutation (8) entre le signal de commande (I) et le moment auquel la position d'extrémité de la soupape de commutation (8) comme étant la durée ($\Delta t$).

7. Procédé selon l'une quelconque des revendications précédentes, **caractérise en ce que** l'on retrouve directement la viscosité ($\eta$) du liquide hydraulique à l'aide la durée ($\Delta t$), sur la base d'une association connue.

8. Procédé selon la revendication 7, **caractérisé en ce que** l'association entre la durée ($\Delta t$) et la viscosité ($\eta$) se produit par régression linéaire.

9. Procédé selon l'une quelconque des revendications précédentes, **caractérisé en ce que** la durée ($\Delta t$) est déterminée de façon répétée à un point de travail défini, notamment à une température (T) définie du liquide hydraulique et que l'on vérifie l'absence ou la présence d'un changement.

10. Procédé selon l'une quelconque des revendications précédentes, **caractérisé en ce qu'**on déduit un vieillissement du liquide hydraulique à l'aide de la durée ($\Delta t$) à un point de travail défini.

11. Procédé selon l'une quelconque des revendications précédentes, **caractérisé en ce que** la grandeur caractéristique de viscosité ($\eta$) déterminée est analysée et déduite au choix ou en combinaison pour :

    - une détermination d'une mesure pour une quantité d'huile ;
    - une détection d'huiles moteur non admises ;
    - une détermination d' un intervalle de vidange d'huile ;
    - un diagnostic du composant hydraulique (8)
    - la commande de soupape.

12. Dispositif de commande (20) pour la détermination d'une grandeur caractéristique de viscosité ($\eta$) d'un liquide hydraulique dans une machine équipée d'une commande hydraulique, notamment d'une huile moteur dans un véhicule automobile équipé d'une commande hydraulique des soupapes d'échange de gaz, un composant hydraulique (8) étant déplacé d'une première position dans une deuxième position à l'aide d'un signal de commande (I), le dispositif de commande (20) étant réalisé de telle sorte qu'une durée ($\Delta t$) nécessaire pour faire passer le composant (8) de la première dans la deuxième position est déduite, cette durée servant de mesure pour la grandeur caractéristique de viscosité ($\eta$), **caractérisé en ce que** l'on déduit de façon complémentaire à l'aide de la durée ($\Delta t$) pour atteindre le point de travail un vieillissement du composant hydraulique.

**FIG 1**

**FIG 2**

FIG 3

FIG 4

FIG 5

FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1544419 A1
- JP 2004092593 B
- US 20040000288 A1
- US 20040244742 A1
- DE 19741164 A1
- WO 03103837 A1
- EP 1533506 A2